# EUROPEAN PATENT SPECIFICATION

(11) **EP 4 178 639 B1**
(45) Date of publication and mention of the grant of the patent: **23.09.2026**
(21) Application number: 21745377.8
(22) Date of filing: 01.07.2021
(51) Int. Cl.: A61L 31/12, A61B 17/00, A61K 51/12, A61L 31/18, B32B 5/00, A61B 17/58, A61B 17/72, A61B 17/80, A61B 17/86, B32B 1/00, B32B 3/26, B32B 5/02, B32B 5/12, C08L 71/00

(54) **COMPOSITE MATERIALS**
VERBUNDWERKSTOFFE
MATÉRIAUX COMPOSITES

(30) Priority: 09.07.2020 GB 202010576
(43) Date of publication of application: 17.05.2023
(73) Proprietor: Invibio Device Component Manufacturing Limited, Thornton Cleveleys, Lancashire FY5 4QD (GB)
(72) Inventor: PATEL, Dharmesh, Cleveleys Lancashire FY5 4QD (GB); LAMORINIERE, Steven, Cleveleys Lancashire FY5 4QD (GB)
(74) Representative: Thompson, Nicola Ruth
(86) International application number: PCT/GB2021/051670
(87) International publication number: WO 2022/008877

(56) References cited:
- WO-A1-2016/035010
- WO-A1-2017/029476
- LUO HONGLIN ET AL: "Preparation of three-dimensional braided carbon fiber-reinforced PEEK composites for potential load-bearing bone fixations. Part I. Mechanical properties and cytocompatibility", JOURNAL OF THE MECHANICAL BEHAVIOR OF BIOMEDICAL MATERIALS, ELSEVIER, AMSTERDAM, NL, vol. 29, 17 September 2013 (2013-09-17), pages 103 - 113, XP028783885, ISSN: 1751-6161, DOI: 10.1016/J.JMBBM.2013.09.003
- HUANG Z M ET AL: "Stiffness and strength design of composite bone plates", COMPOSITES SCIENCE AND TECHNOLOGY, ELSEVIER, AMSTERDAM, NL, vol. 65, no. 1, 1 January 2005 (2005-01-01), pages 73 - 85, XP027688077, ISSN: 0266-3538, [retrieved on 20050101]

## Description

### BACKGROUND

The present invention relates to a composite material. The present invention also relates to a device, for example, a medical device comprising the composite material of the present invention.

Composite materials comprising polymer and reinforcement fibres are known. For example, carbon fibre has been used as a reinforcement fibre in various polymeric composite materials.

Such composite materials may be used in a range of applications, including, for example, in the manufacture of medical devices. For example, such composite materials may be used to make implantable devices, such as orthopaedic implants.

WO 2016/035010 A1 describes composite material bone implant devices.

WO 2017/029476 A1 describes an implantable medical device having a body comprising a composite material, and a method of manufacturing such a device.

LUO HONGLIN ET AL, JOURNAL OF THE MECHANICAL BEHAVIOR OF BIOMEDICAL MATERIALS, vol. 29, 17 September 2013, p 103-113 describes fabrication and characterization of three-dimensional carbon fiber-reinforced polyetheretherketone (C_{3-D}/PEEK) composites for orthopedic applications.

HUANG Z M ET AL, COMPOSITES SCIENCE AND TECHNOLOGY, vol. 65, no. 1, 1 January 2005, p 73-85 describes a preliminary design procedure for composite bone plate with target on both its stiffness and its ultimate strength.

It is among the objects of embodiments of the invention to provide a composite material with an improved balance of mechanical properties. It is also among the objects of embodiments of the invention to provide a composite material that has improved mechanical compatibility with biological materials, for example, bone.

### BRIEF DESCRIPTION OF THE DRAWINGS

Embodiments of the present invention are described, by way of example, with reference to the following figures, in which:
Figure 1 is a schematic drawing of a lay-up arrangement that may be used in the manufacture of an implantable device by compression moulding;
Figure 2 is an example of a fracture plate that may be manufactured using a composite material of an embodiment of the present invention;
Figure 3 is a bar chart showing the maximum torque that can be subjected to fracture plates manufactured using the composite materials of Example 1 (see Example 2);
Figure 4 is a bar chart showing the extent of screw rotation at maximum torque in fracture plates manufactured using the composite materials of Example 1 (see Example 2);
Figure 5 is a bar chart showing the extent of screw rotation at functional torque in fracture plates manufactured using the composite materials of Example 1 (see Example 2);
Figure 6 is a bar chart showing the bending strength exhibited by fracture plates manufactured using the composite materials of Example 1 (see Example 3);
Figure 7 is a bar chart showing the bending structural stiffness exhibited by fracture plates manufactured using the composite materials of Example 1 (see Example 5);
Figure 8 is a bar chart showing maximum load exhibited by fracture plates using composite materials of Example 1; and
Figure 9 is a bar chart showing flexural modulus exhibited by fracture plates manufactured using composite materials of Example 1.

### DESCRIPTION

According to one aspect of the present invention, there is provided a composite material comprising polyaryletherketone, reinforcement fibre, and contrast agent, wherein the contrast agent comprises barium, and wherein the reinforcement fibre is present in an amount of 52 to 58 volume % based on the total volume of the composite material.

The contrast agent may be an X-ray detectable material. For example, the contrast agent may be barium sulphate.

Preferably, the reinforcement fibre is carbon fibre.

Preferably, the polyaryletherketone is polyetheretherketone (PEEK).

The composite material may be provided in the form of a tape or sheet. Sheets may be formed of joined portions of tape. The composite material may take the form of a net or other precursor that can be used to form a 3-D object of a desired shape and/or configuration.

The composite material may be used to form a device.

In yet a further aspect, there is provided a method of manufacturing a device, said method comprising compression-moulding layers of composite material together to form a body portion of the device, wherein at least one layer comprises a composite material as described herein.

The device may be a medical device, for example, an implantable device. Examples of implantable devices include orthopaedic implants, for instance, fracture plates and trauma plates, intramedullary nails, spinal implants such as cages, rods and screws, and other load bearing implantable devices.

In the case of a medical device, contrast agent may not be present in the composite material. For example, the contrast agent may be applied separately. Accordingly, in a further aspect, the present invention provides a medical device comprising a composite material that comprises polyaryletherketone, and reinforcement fibre, wherein the reinforcement fibre is present in an amount of 52 to 58 volume % based on the total volume of the composite material.

It has been found that, when the amount of reinforcement fibre (carbon fibre) in the composite material is 52 to 58 volume %, the composite material provides an improved balance of mechanical properties. For example, the flexibility and/or mouldability of the composite material may be enhanced without overly compromising on strength. This is surprising, since reinforcement fibres are generally used to increase both the strength and stiffness of materials. In some embodiments, therefore, the composite material can provide a desirable degree of stiffness without overly compromising on strength.

Advantageously, the composite material of the present invention may also have improved mechanical compatibility with biological materials, such as bone. Accordingly, when an implant formed from the composite material of the present invention is implanted into bone, stress-shielding may be reduced and the distribution of load improved. This can provide the underlying bone with sufficient biological stimulus for maintaining bone mass and/or reducing bone loss.

Furthermore, when the composite material of the present invention is used to manufacture an implantable device, it has been found that the implantable device is resistant to damage e.g. relative to other variants of similar composites even when relatively high insertion torques are applied to screw(s) used to secure the implant. Moreover, the mechanical properties of the composite material are such that, the extent to which the screw(s) rotate upon application of typical insertion torques is sufficient to reduce the risk of failure from over-tightening during implantation.

### Polyaryletherketone

Any suitable polyaryletherketone may be used in the composite material of the present invention.

Suitable polyaryl ether ketone may have repeating units of formula (I) below: 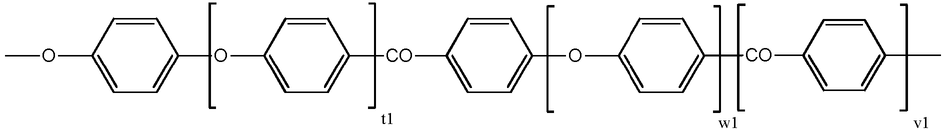 where t1 and w1 are independently represent 0 or 1 and v1 represents 0, 1 or 2.

The polyaryletherketone suitably includes at least 90, 95 or 99 mol % of repeat unit of formula I.

The polyaryletherketone may comprise or consist essentially of a repeat unit of formula I. Preferred polymeric materials comprise (or consist essentially of) a said repeat unit wherein t1=1 , v1=0 and w1=0; t1=0, v1=0 and w1=0; t1=0, w1 =1 , v1=2; or t1=0, v1=1 and w1=0. More preferably, the polyaryletherketone comprises (e.g. consists essentially of) the repeat unit I, wherein t1 =1 , v1 =0 and w1 =0; or t1=0, v1=0 and w1 =0. The most preferred polyaryletherketone comprises (especially consists essentially of) a said repeat unit wherein t1=1 , v1=0 and w1=0.

In preferred embodiments, the polyaryletherketone is selected from polyetheretherketone, polyetherketone, polyetherketoneetherketoneketone and polyetherketoneketone.

In a more preferred embodiment, the polyaryletherketone is polyether ether ketone or PEEK.

In some examples, the polyaryletherketone (e.g. PEEK) may have a Notched Izod Impact Strength (specimen 80mm x 10mm x 4mm with a cut 0.25mm notch (Type A), tested at 23°C, in accordance with ISO180) of at least 4 KJmT⁻², preferably at least 5 KJmT⁻², more preferably at least 6 KJmT⁻². The Notched Izod Impact Strength, measured as described above, may be less than 10 KJmT⁻², suitably less than 8 KJmT⁻². The Notched Izod Impact Strength, measured as described above, may be at least 3 KJmT⁻², suitably at least 4 KJmT⁻², preferably at least 5 KJmT⁻². The Notched Izod Impact Strength may be less than 50 KJmT⁻², suitably less than 30 KJmT⁻².

The polyaryletherketone (e.g. PEEK) suitably has a melt viscosity (MV) of at least 0.06 kNsm⁻², preferably has a MV of at least 0.09 kNsm⁻², more preferably at least 0.12 kNsm⁻². The polyaryletherketone (e.g. PEEK) may have a MV of less than 1.00 kNsm⁻², preferably less than 0.5 kNsm⁻².

The polyaryletherketone (e.g. PEEK) may have a MV in the range 0.09 to 0.5 kNsm⁻², preferably in the range 0.1 to 0.3 kNsm⁻², preferably having a MV in the range 0.1 to 0.2 kNsm⁻². An MV of 0.15 kNsm⁻² has been found to be particularly advantageous. MV is suitably measured using capillary rheometry operating at 400°C at a shear rate of 1000s⁻¹ using a tungsten carbide die, 0.5mm x 3.175 mm.

In a preferred embodiment, the polyaryletherketone (e.g. PEEK) has a melt viscosity (MV) of 0.09 kNsm⁻² to 0.5 kNsm⁻².

The polyaryletherketone (e.g. PEEK) may be amorphous or semi-crystalline. The polyaryletherketone is preferably crystallizable. The polyaryletherketone is preferably semi-crystalline. The level and extent of crystallinity in a polymer is preferably measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calorimetry (DSC).

The level of crystallinity of said polyaryletherketone (e.g. PEEK) may be at least 1 %, suitably at least 3%, preferably at least 5% and more preferably at least 10%. In especially preferred embodiments, the crystallinity may be greater than 25%. It may be less than 50% or less than 40%.

The main peak of the melting endotherm (Tm) of the polyaryletherketone (if crystalline) may be at least 300°C. Where e.g. PEEK is used, the main peak of the melting endotherm (Tm) may be at least 300°C.

The composite material may comprise any suitable amount of the polyaryletherketone (e.g. PEEK). For example, the composite material may comprise at least 20 volume %, preferably at least 25 volume %, more preferably at least 30 volume %, yet more preferably at least 35 volume %, even more preferably at least 37 volume % and most preferably at least 39 volume % polyaryletherketone (e.g. PEEK). The composite material comprises up to 48 volume % polyaryletherketone (e.g. PEEK). In some embodiments, the composite material may comprise up to 45 volume %, up to 43 volume % polyaryletherketone (e.g. PEEK).

In some embodiments, the composite material may comprise 20 to 48 volume %, preferably 30 to 48 volume %, more preferably 35 to 48 volume %, yet more preferably 37 to 48 volume % or 38 to 48 volume % polyaryletherketone (e.g. PEEK). More preferably, the composite material may comprise 39 to 48 volume %, even more preferably 39 to 45 volume % polyaryletherketone (e.g. PEEK). In some embodiments, the composite material may comprise 39 to 43 volume % polyaryletherketone (e.g. PEEK).

The volume ratio of reinforcement fibre to polyaryletherketone (e.g. PEEK) is 1.1 : 1 to 1.5 : 1, for example, 1.2 : 1 to 1:4 :1.

### Reinforcement fibres

Any suitable reinforcement fibre may be used. The fibres used may be selected from inorganic or organic fibrous materials. The fibres may have a melting or decomposition temperature of greater than 200 °C, for example, greater than 250 °C or greater than 300 °C. In some embodiments, the fibres may have a melting temperature of greater than 350 °C or 500 °C. Examples of suitable fibres include aramid fibres, carbon fibre, glass fibre, carbon fibre, silica fibre, zirconia fibre, silicon nitride fibre, boron fibre and potassium titanate fibre. Most preferred fibres are carbon fibres.

The reinforcement fibre (e.g. carbon fibre) may have a tensile strength of greater than 4200 MPa, preferably greater than 4500 MPa, more preferably greater than 4800 MPa..

The reinforcement fibre (e.g. carbon fibre) may have a tensile modulus of greater than 200 GPa, preferably greater than 230 GPa, more preferably greater than 240 GPa.

The reinforcement fibre (e.g. carbon fibre) may have a strain at failure of greater than 1.1%, preferably, greater than 1.2%, 1.4% or 1.6% The reinforcement fibre (e.g. carbon fibre) may have a strain at failure of less than 2.2%, for instance, less than 2.0% or 1.9%. In some embodiments, reinforcement fibre (e.g. carbon fibre) may have a strain at failure of 1.2 to 2.2%, for example, 1.4 to 2.0 % or 1.6 to 1.9%. In one embodiment, the reinforcement fibre (e.g. carbon fibre) may have a strain at failure of 1.7 to 1.9%.

The reinforcement fibre (e.g. carbon fibre) may have a mass per unit length of 0.1 to 1.0 g/m, for example, 0.2 to 0.8 g/m. In some embodiments, the 0.2 to 0.5 g/m.

The reinforcement fibre (e.g. carbon fibre) may have a density of greater than 1.65 g/cm³, preferably greater than 1.70 g/cm³. The reinforcement fibre (e.g. carbon fibre) may have a density of less than 1.85 g/cm³, preferably less than 1.80 g/cm³. In some embodiments, the reinforcement fibre (e.g. carbon fibre) may have a density of 1.70 to 1.85 g/cm³, for example, 1.75 to 1.80 g/cm³, or 1.78 to 1.79 g/cm³.

The reinforcement fibre (e.g. carbon fibre) may be provided in the form of a continuous tow. Any suitable tow size may be used. The tow size indicates the number of filaments in the tow. In some embodiments, the tow size may be 1000 to 24,000. In one embodiment, a tow size of 6000 to 12,000 may be employed.

Examples of suitable reinforcement fibre include carbon fibres supplied, for example, by Hexcel^{®} under the trademark HexTow^{®}.

The reinforcement fibre (e.g. carbon fibre) may be present in an amount of 52 to 58 volume % based on the total volume of the composite material. Preferably, the reinforcement fibre (e.g. carbon fibre) may be present in an amount may be 54 to 56 volume %. In one embodiment, the reinforcement fibre (e.g. carbon fibre) may be present in an amount of 55 volume %.

As discussed above, the amount of reinforcement fibre (e.g. carbon fibre) in the composite material can be controlled within a narrow range to enable the composite material to provide an optimised balance of mechanical properties. For example, the flexibility of the composite material may be enhanced without compromising on strength. This is surprising, since reinforcement fibres are generally used to increase both the strength and stiffness of materials.

Advantageously, the amount of reinforcement fibre (e.g. carbon fibre) is also controlled within a narrow range to improve the mechanical compatibility of the composite material with biological materials, such as bone. Accordingly, when an implant formed from the composite material of the present invention is implanted into bone, stress-shielding may be reduced and the distribution of load improved. This can ensure that the bone is provided with sufficient biological stimulus for maintaining bone mass and/or reducing bone loss.

Furthermore, the amount of reinforcement fibre (e.g. carbon fibre) is also controlled within a narrow range so that, when the composite material of the present invention is used to manufacture an implantable device, the mechanical properties of the device are controlled such that it is resistant to damage even when relatively high insertion torques are applied to fixation screw(s) used to secure the implant. Moreover, the mechanical properties of the composite material are such that, the extent to which the fixation screw(s) rotate upon application of typical insertion torques is sufficient to reduce the risk of over-tightening during implantation.

In the invention, the composite material also comprises a contrast agent wherein the contrast agent comprises barium, e.g. barium sulphate. For example, barium sulphate may be present in the composite material in an amount of 2 to 20 weight % of the total weight of the composite material, for instance, 3 to 10 weight %. In a preferred embodiment, the amount of barium sulphate may be 4 to 8 weight %, more preferably 4 to 6 weight %. In a most preferred embodiment, the amount of barium sulphate may be 5 weight %.

It has been found that, by using controlled amounts of the reinforcement fibre (e.g. carbon fibre) in combination with contrast agent, e.g. barium sulphate, the properties of the composite material may be further improved. For example, by controlling the amount of reinforcement fibre (e.g. carbon fibre) in the presence of barium sulphate, it has been found that the extent to which the screw(s) rotate upon application of typical insertion torques can be increased. This may be advantageous because the surgeon may be provided with a greater degree of tactile feedback during the implantation process, creating a greater perception of screw insertion and consequently reducing the risk of over-tightening. During implantation, it is generally desirable to ensure that the fixation screw(s) do not stand proud of the implant. However, in many instances, the desire to avoid protruding screw(s) results in over-tightening of the screws, placing the device and underlying bone under undue stress. The risk of over-tightening may be increased when the fixation screw(s) are resistant to rotation, as this may encourage the surgeon to apply greater torques than necessary. By tailoring the mechanical properties of the composite material, the extent of rotation can be controlled, so as to improve feedback between the implantable device and the surgeon during implantation.

By using controlled amounts of the reinforcement fibre (e.g. carbon fibre) in combination with contrast agent, e.g. barium sulphate, it may also be possible to vary the properties of the composite material in terms of its imageability under e.g. X-ray. For example, while barium sulphate may provide sufficient radio-opacity for an implantable device to be detected under e.g. X-ray, the amount of reinforcement fibre (e.g. carbon fibre) is controlled within narrow limits to provide or maintain sufficient translucency to allow the fracture in the underlying bone to be detected under imaging techniques e.g. X-ray.

Moreover, by using controlled amounts of the reinforcement fibre (e.g. carbon fibre) in combination with barium sulphate, the radio-translucency of the composite material may be optimized to reduce interference, such that dosing accuracy during radiotherapy can be maintained.

### Contrast Agent

In the present invention, the contrast agent comprises barium. Preferably, the contrast agent is detectable by X-ray. For instance, the contrast agent may be barium sulphate.

Barium sulphate is a contrast medium that allows the composite material to be detected under imaging techniques, for example, X-ray. Accordingly, when the composite material is used in the manufacture of an implantable device, the device may be detected under e.g. X-ray.

The barium sulphate may have a D₁₀ particle size in the range of 0.1 to 1.0 microns; a D₅₀ particle size in the range of 0.5 to 2.0 microns and a D₉₀ particle size in the range of 1.0 to 5 microns. The D₁₀ particle size may be in the range of 0.1 to 0.6 microns, preferably 0.2 to 5 microns. The D₅₀ particle size may be in the range of 0.7 to 1.5 microns, preferably 0.8 to 1.3 microns. The D₉₀ particle size may be in the range of 1.5 to 3 microns, preferably in the range of 2.0 to 2.5 microns.

Suitable X-ray grade barium sulphate may be available from Merck-Millipore^{®}.

Any suitable amount of contrast agent e.g. barium sulphate may be used. For example, contrast agent e.g. barium sulphate may be present in the composite material in an amount of 2 to 20 weight %, preferably, 3 to 15 weight %, for instance, 3 to 10 weight %. In a preferred embodiment, the amount of contrast agent e.g. barium sulphate may be 3 to 8 weight %, more preferably 3 to 5 or 4 to 6 weight %. In a most preferred embodiment, the amount of contrast agent e.g. barium sulphate may be 5 weight %.

The amount of contrast agent e.g. barium sulphate may be controlled, such that the radio-translucency of the composite material is optimized to reduce interference. This can allow dosing accuracy during radiotherapy to be maintained.

As noted above, the relative amounts of reinforcement fibre (e.g. carbon fibre) and e.g. barium sulphate may be controlled to improve the properties of the composite material. In particular, by controlling the relative amounts of reinforcement fibre (e.g. carbon fibre) and e.g. barium sulphate, it has been found that the extent to which the fixation screw(s) rotate upon application of typical insertion torques can be increased. This may be advantageous because the surgeon may be provided with a greater degree of feedback during the implantation process, reducing the risk of over-tightening. During implantation, it is desirable to ensure that the fixation screw(s) do not stand proud of the implant. However, in many instances, the desire to avoid protruding fixation screw(s) results in over-tightening of the screws, placing the device and underlying bone under undue stress. The risk of over-tightening may be increased when the fixation screw(s) are resistant to rotation, as this may encourage the surgeon to apply greater insertion torques than necessary. By tailoring the mechanical properties of the composite material, the extent of rotation can be controlled, so as to improve feedback between the implantable device and the surgeon during implantation.

Furthermore, by controlling the relative amounts of the reinforcement fibre (e.g. carbon fibre) to barium sulphate, it may also be possible to vary the properties of the composite material in terms of its imageability under e.g. X-ray. For example, the relative amounts of the reinforcement fibre (e.g. carbon fibre) to barium sulphate may be controlled to allow the implantable device to be detected under e.g. X-ray, while maintaining sufficient translucency to allow the fracture in the underlying bone to be detected.

Moreover, the relative amounts of the reinforcement fibre (e.g. carbon fibre) to barium sulphate may be controlled, such that the radio-translucency of the composite material is optimized to reduce interference. This can allow dosing accuracy during radiotherapy to be maintained.

### Composite Material

As noted above, the composite material of the present invention comprises polyaryletherketone, and reinforcement fibre, wherein the reinforcement fibre is present in an amount of 52 to 58 volume % based on the total volume of the composite material.

The composite material may be formed as a sheet or tape. For example, the reinforcement fibre (e.g. carbon fibre) may be combined with the polyaryletherketone (e.g. PEEK) and formed into a tape or sheet. The tape or sheet may be formed, for example, using heat and/or compression. In an embodiment, the polyaryletherketone (e.g. PEEK) may be heated to above its softening or melting temperature to melt or soften the polymer around the fibres to form the composite. The molten or soften polymer is then compressed around the fibres to form the sheet or tape.

When heat is applied, suitable temperatures include temperatures of 320°C and above, preferably, of 330°C and above, more preferably, of 340°C and above. In some embodiments, compression moulding may be carried out at temperatures of 320 to 450°C, preferably 330 to 400°C, more preferably 340 to 380 °C and yet more preferably 350 to 370°C. Suitably, pressures of at least 1.5 MPa or at least 2 MPa may be applied. Examples of suitable pressures range from 1.5 to 10 MPa, for instance, 2 to 8 MPa.

The tape or sheet formed using the composite material of the present invention may have a thickness of 10 microns to 1 mm, preferably 100 to 300 microns, more preferably 140 to 200 microns.

The tape or sheet may be used to form a device, for example, a medical device as explained below.

### Device

As discussed above, an aspect of the present invention relates to a device comprising the composite material described herein. Another aspect of the present invention relates to a method or manufacturing a device, which comprises compression-moulding layers of composite material together to form a body portion of the device, wherein at least one layer comprises a composite material as described herein.

The device may be made from a tape or sheet comprising the composite material. For example, a tape or sheet may form at least one of the layers of composite material that are compression-moulded together to form a body portion of the device.

Where tape is employed to form a layer, multiple tapes may be aligned unidirectionally in the layer. Multiple layers of tape may then be overlaid in a lay-up arrangement, and compression moulded to form a laminate, for example, in the shape of the body of the device.

In some embodiments, the tape in a first layer may be aligned unidirectionally along an axis. Tape in a second layer may be aligned unidirectionally at an angle to the axis of the first layer. In the third layer of the lay-up, the tape may be unidirectionally aligned at an angle that is different from the angle of the second layer and/or the first layer. These and subsequent layers may be oriented at angles depending on the desired properties and/or shape of the device. The lay-up arrangement may be determined using computer software, depending on the shape, configuration and/or properties of the final device.

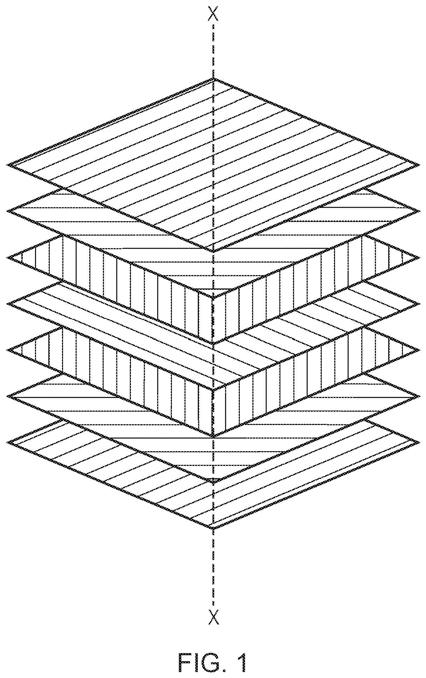

In one example, the tape in the first layer are aligned at 0° to the axis. The tape in the second layer may be aligned at 90° to the axis, while the third and fourth layers may be aligned at 0° and 90° to the axis, respectively. This alternating pattern may be continued throughout the laminate. In an alternative embodiment, the tape in the first layer are aligned at 0° to the axis. The tape in the second layer may be aligned at 45° to the axis, while the third layer may be aligned at 90° to the axis. The fourth layer may be aligned at -45 ° to the axis, while the fifth layer may be aligned at 0° to the axis and so on. Figure 1 illustrates an example of a suitable lay-up for producing a laminate for the device.

The tape in the lay-up arrangement may be compression moulded to form the device or part thereof. Compression moulding may be carried out by applying heat and pressure. The resulting part may then be cooled, for example, under pressure.

When heat is applied, suitable temperatures include temperatures of 320°C and above, preferably, of 330°C and above, more preferably, of 340°C and above. In some embodiments, compression moulding may be carried out at temperatures of 320 to 450°C, preferably 330 to 400°C, more preferably 340 to 380 °C and yet more preferably 350 to 370°C. Suitably, pressures of at least 1.5 MPa or at least 2 MPa may be applied. Examples of suitable pressures range from 1.5 to 10 MPa, for instance, 2 to 8 MPa.

In some embodiments, after heat and pressure are applied, the part is rapidly cooled, preferably to between 140°C and 200°C. In some embodiments, cooling may be carried out under pressure.

Where a plurality of layers are employed, at least one of the layers may comprise the composite material of the present disclosure. The at least one layer may be formed of e.g. tapes or a sheet formed from a composite material of the present disclosure. In some embodiments, at least 10% of the layers employed to make the device comprise a composite material of the present disclosure. In some embodiments, at least 20%, preferably at least 30%, more preferably at least 50%, yet more preferably at least 60%, even more preferably at least 60%, yet more preferably at least 70% of the layers employed to make the device comprise a composite material of the present disclosure. In some examples, at least 70%, preferably at least 80% and more preferably at least 90% of the layers employed to make the device comprise a composite material of the present disclosure. In a preferred embodiment, at least 95% of the layers employed to make the device comprise a composite material of the present disclosure. In a most preferred embodiment, all the layers consist essentially of a composite material of the present disclosure.

While the same composite material may be used in all of the layers, it may also be possible to use different composite materials in each of the layers. Where different composite materials are employed, the composite materials may differ in terms of, for example, their barium sulphate content. Additionally or alternatively, they may differ in terms of their content of reinforcement fibres (e.g. carbon fibre) and/or polyaryletherketone (e.g. PEEK).

The composite material in each of the layers may be an embodiment of the present invention. For example, the layers may comprise two or more embodiments of the present invention. Alternatively, the composite material in each of the layers may be formed of the same embodiment of the present invention. In yet another alternative embodiment, some of the layers may comprise composite material of the present invention, while other layers may comprise composite material that fall outside the scope of the present invention. Even the layers comprising composite material falling outside the scope of the present invention, however, may comprise polyaryletherketone (e.g. PEEK). Such composite material may also comprise reinforcement fibre (e.g. carbon fibre) and/or barium sulphate.

The composition of the layers in the laminate may be varied depending on the shape, configuration and/or properties of the relevant parts of the device.

In some examples, the layers comprise the same polyaryletherketone (e.g. PEEK).

Some of the layers of the device may be free from barium sulphate, while some of the layers may comprise barium sulphate.

In a preferred embodiment, at least an outer region (e.g. outer or external surface) of the device comprises barium sulphate. This can allow the outer region of the device to be detectable using imaging techniques, for example, X-ray. In some instances, an inner region of the device may comprise barium sulphate. In some instances, barium sulphate may be present throughout the device.

The outer region may be formed of a layer formed, for example, of a sheet or tapes of composite material.

Where the device has a body having a variable cross-section, the device may be formed using the method described in WO 2017/029476. For example, the body may be formed of a first external layer and a second external layer. Packing layers may be positioned between the first external layer and second external layer, and an insert layer(s) may be positioned within the packing layers to provide increased depth to a portion of the body. The layers may be compression moulded together to form the body of the device. The first external layer may contact the second external layer, so as to reduce exposure of the ends of the packing layers and insert layer(s), thereby reducing the risk of delamination of the moulded part. As discussed above, any one of the external, packing or insert layers may be a composite material of the present disclosure.

Any suitable device may be made from the composite material of the present disclosure. For example, the device may be a medical device. As explained above, the composite material used to make the medical device may contain a contrast agent e.g. barium sulphate, or may be free from contrast agent. Preferably, the composite material used to make the medical device comprises contrast agent (e.g. barium sulphate). Preferably, the device may be an implantable device. Examples include orthopaedic implants, for instance, fracture plates and/or trauma plates, intramedullary nails, spinal implants such as cages, rods and screws, and other load bearing or bone contacting implants. In a preferred embodiment, the device is a fracture plate or trauma plate.

The device may include one or more apertures. For example, the device may include a threaded aperture e.g. for receiving a fixation screw for securing the device to an underlying structure. In the case of an orthopaedic implant, the device may include a threaded aperture for receiving a screw for securing the device to underlying bone. In one embodiment, the device is an implantable implant comprising one or more threaded apertures. In a preferred embodiment, the device may be a fracture plate or trauma plate comprising one or more threaded apertures. As discussed above, the composite material of the present disclosure can be used to produce an implant that has advantages, for example, when an insertion torque is applied to a screw used to secure the implant to underlying bone. The implant may be resistant to damage on application of relatively high insertion torques. Moreover, as discussed above, in some embodiments, the composite material of the present disclosure may be used to produce implants that provide a surgeon with a greater degree of feedback during the implantation process, reducing the risk of over-tightening.

These and other aspects of the present invention will now be described with reference to the accompanying drawings.

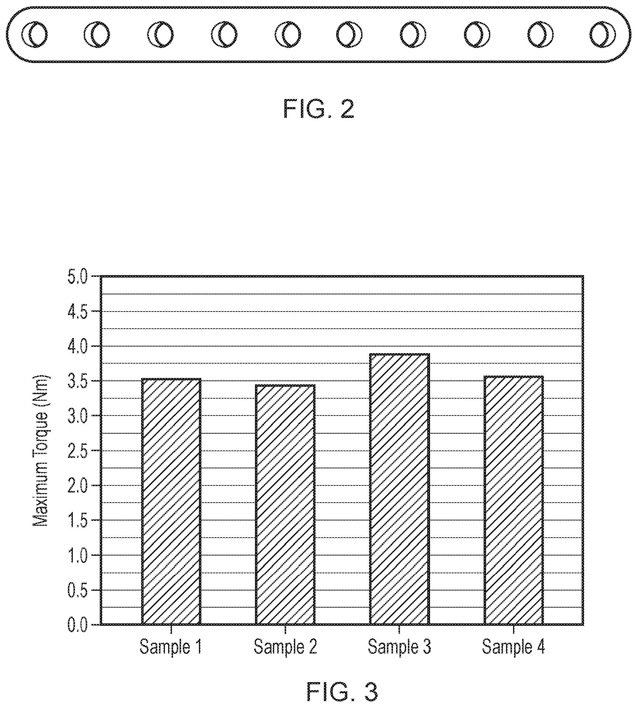

Referring to Figure 1, this is a schematic drawing of a lay-up arrangement of layers of tape formed from the composite material of an embodiment of the present invention. Starting from the bottom of the lay-up as shown, the first layer is formed of tape that is unidirectionally aligned along an axis (0°). The second layer is formed of tape that is unidirectionally aligned at 45° to the axis of the first layer. The third layer is formed of tape 14c that is unidirectionally aligned at -45° to the axis of the first layer. The fourth layer is formed of tape 14d that is unidirectionally aligned at 90° to the axis of the first layer. The pattern is repeated so that the overall structure has the following alignment: 0°, 45°, -45°, 90°, -45°, 45° and 0. The resulting laminate may be compression moulded under heat and pressure to form a device, for example, the fracture plate shown in Figure 2.

The fracture plate of Figure 2 comprises a plurality of threaded apertures. The threaded apertures are configured for receiving locking screws, for example, 3.5 mm locking screws.

### Example 1

In this Example, composite materials having the compositions shown in Table 1 were compression moulded to form a fracture plate as shown schematically in Figure 2. The plates formed were 3.2 mm thick.

**Table 1**

| Sample | Carbon Fibre (vol %) | BaSO₄ (weight %) |
|---|---|---|
| 1 | 55 | 5 |
| 2 | 55 | 0 |
| 3 | 58 | 0 |
| 4 (Reference) | 62 | 0 |
| 5 | 55 | 4 |
| 6 | 55 | 6 |

### Example 2

### Torque Tests

Torque tests were carried out on an MTS Bionix EM Torsion System with a 10 Nm load cell. Locking screws (3.5 mm) were cleaned via an ultrasonic wash in distilled water prior to conducting the torque tests. For each torque test, a screw was inserted into the end hole of a plate by hand using a torque limiting driver set to 0.1 Nm. The screw and plate assembly were then placed into an MTS Bionix EM Torsion system, aligned with the driver bit and then clamped into place. A compressive axial force of 1 kg was applied, and the driver bit rotated at 1 RPM in the same direction of screw insertion. Specimens were tested to failure at 1 RPM whilst torque and rotation data were collected at 100Hz. The maximum torque, rotation at maximum torque and rotation at 1.5 Nm (i.e. locking torque) were extracted from the data.

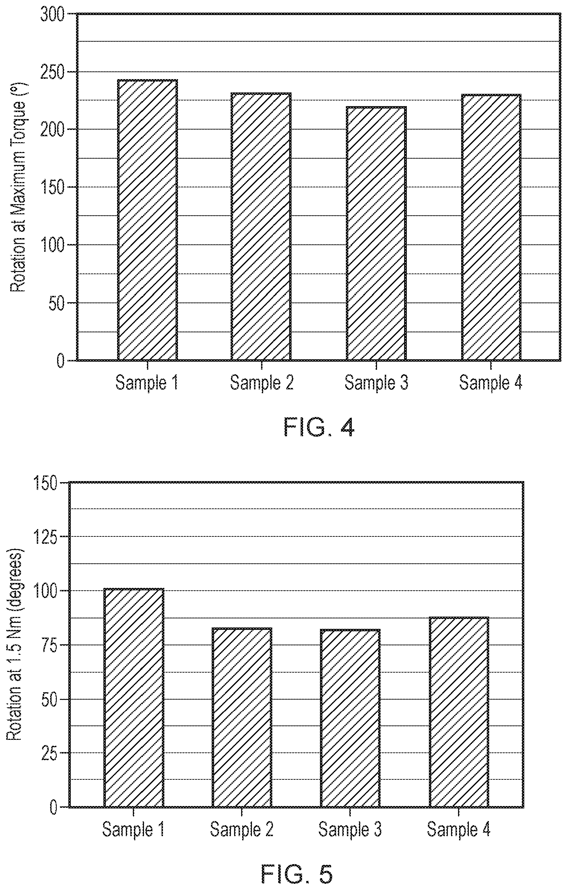

The results are shown in the bar graphs in Figures 3, 4 and 5. Fig. 3 shows that all samples could be subjected to torques (maximum torques) that far exceeded the functional insertion torque of 1.5 Nm typically required to insert the screws used in the tests. Figure 4 shows that the screws inserted into all samples rotated by comparable amounts when subjected to maximum torque, with sample 1 exhibiting the greatest screw rotation. Figure 5 shows that at a functional torque of 1.5 Nm, sample 1 exhibited the greatest screw rotation. Greater rotation may be advantageous because the surgeon may be provided with a greater degree of tactile feedback during the implantation process, creating a greater perception of screw insertion and consequently reducing the risk of over-tightening.

### Example 3

### Flexural tests - 4-point bend

Flexural tests were conducted on the Sample of Example 1 in a four-point bend setup following the principles from ASTM F382-17: Standard Specification and Test Method for Metallic Bone Plates.

Tests were conducted on an Instron 3345 test machine with a 1 kN load cell and uniform cylindrical (10 mm diameter) support and loading rollers. The support and loading rollers were positioned to create one-third loading points between support rollers. The loading and centre span were both set to 26 mm which allowed the specimens to be positioned such that two holes were present between the loading rollers as well as two holes between each loading roller and the adjacent support roller. A compressive axial 2 N pre-load was then applied to remove slack, after which the specimens were tested to failure at a rate of 2 mm/min. Load and displacement data were collected during the test at 100Hz and Failure was defined as a 20 % drop in load.

The bending structural stiffness and bending strength were calculated using the following formulae:
Bending stiffness (K) (N/mm) = Slope of the initial linear-elastic region of the load vs displacement curve $\begin{matrix}Bending structural stiffness \left({Nm}^{2}\right) = \\ \frac{\left(2h+3a\right) {Kh}^{2}}{12}\end{matrix}$ $\begin{matrix}Bending strength \left(Nm\right) = \\ \frac{Ph}{12}\end{matrix}$ Where:
h = Loading span (mm)
a = Centre span (mm)
K = Bending stiffness (N/mm)
P = Proof or max load (N)

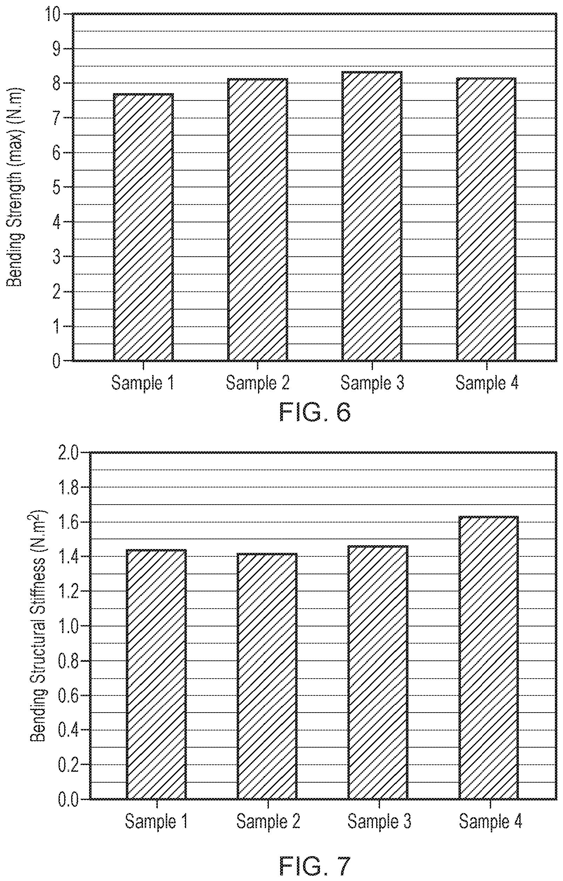

Figures 6 is a bar chart respectively showing the bending structural stiffness of Samples 1 to 4 above. Figures 7 is a bar chart respectively showing the bending strength of Samples 1 to 4 above. Figure 6 shows that comparable bending strengths are achieved across the samples, despite Samples 1 to 3 having lower reinforcement fibre contents than Sample 4. Figure 7 shows that Samples 1 to 3 are more flexible (lower stiffness) than Sample 4. The results depicted in Figures 6 and 7 are surprising as they show that, by decreasing the carbon fibre content, flexibility can be enhanced without unduly compromising on strength.

In addition to the above mentioned advantages, it has also been found that barium sulphate has further positive benefits. Specifically, mechanical stress shielding can lead to bone resorption and a reduction in bone strength (in accordance with Wolff's law). Although known commercial fracture fixation plates possess the flexural strength to withstand loading in situ, disadvantageously, they also exhibit high flexural stiffness creating a large disparity between the bone and plate, resulting in significant stress shielding. In an example arrangement of the present invention, PEEK-OPTIMA^{™} Ultra-Reinforced (by Invibio Limited) having 4-6% barium sulphate is shown to provide sufficient mechanical strength whilst also exhibiting flexural stiffness closer to that of bone to reduce stress shielding. Reducing the flexural modulus of the plate allows the preservation of the maximum flexural load capability (i.e. the maximum load capability is not reduced to the same extent as the flexural modulus).

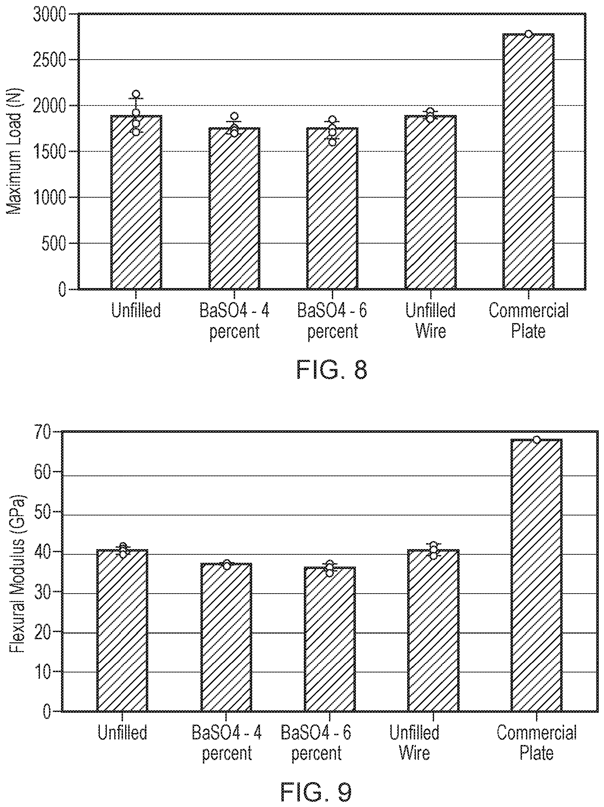

Figure 8 shows that when compared to a commercial polymeric plate (62% carbon fibre, no barium sulphate), a PEEK based plate comprising 55% carbon fibre and 4-6% barium sulphate can retain between 62-68% of the maximum load capability (equivalent to 65-76% maximum flexural stress of the plate) of the commercial plate whilst reducing the bending stiffness to 45-48% (equivalent to 53-60% of the flexural modulus). Moreover, the incorporation of 4-6% barium sulphate showed further reduced flexural stiffness compared to plates without barium sulphate of similar design.

It is known that the modulus for human cortical bone is reported as up to 20 GPa. Therefore, plates with modulus values closer to those of bone whilst also maintaining sufficient loading capability are highly beneficial for preventing stress shielding. Figure 9 shows that plates comprising the composition with contrast agent of the invention have lower flexural modulus compared to plates comprising the said composition without contrast agent, or those of commercial plates comprising 62% fibre content.

The selection of the combination of carbon fibre and barium sulphate is shown to be highly advantageous and will likely promote callus-formation and secondary healing, in addition to barium sulphate enabling visualisation of the plate under x-rays without impeding visualisation of the fracture site, thus allowing fracture healing to be monitored.

### Definitions

It is noted that, as used in this specification and the appended claims, the singular forms "a," "an," and "the" include plural referents unless the content clearly dictates otherwise.

As used herein, the term "about" is used to provide flexibility to a range endpoint by providing that a given value may be "a little above" or "a little below" the endpoint. The degree of flexibility of this term can be dictated by the particular variable and can be determined based on experience and the associated description herein.

As used herein, a plurality of items, structural elements, compositional elements, and/or materials may be presented in a common list for convenience. However, these lists should be construed as though each individual member of the list is individually identified as a separate and unique member. Thus, no individual member of such list should be construed as a *de facto* equivalent of any other member of the same list solely based on their presentation in a common group without indications to the contrary.

Concentrations, dimensions, amounts, and other numerical data may be presented herein in a range format. It is to be understood that such range format is used merely for convenience and brevity and should be interpreted flexibly to include the numerical values explicitly recited as the limits of the range, and also to include all the individual numerical values or sub-ranges encompassed within that range as if the numerical value and sub-range is recited. For example, a weight ratio range of about 1 wt% to about 20 wt% should be interpreted to include the explicitly recited limits of 1 wt% and about 20 wt%, and also to include individual weights such as 2 wt%, 11 wt%, 14 wt%, and sub-ranges such as 10 wt% to 20 wt%, 5 wt% to 15 wt%, etc.

## Claims

1. A composite material comprising
polyaryletherketone,
reinforcement fibre,
and contrast agent, wherein the contrast agent comprises barium, and
wherein the reinforcement fibre is present in an amount of 52 to 58 volume % based on the total volume of the composite material.

2. A composite material as claimed in claim 1, wherein the contrast agent is barium sulphate, and being present in an amount of 3 to 15 weight %.

3. A composite material as claimed any one of the preceding claims, wherein the reinforcement fibre is carbon fibre.

4. A composite material as claimed in any one of the preceding claims, wherein the polyaryl ether ketone is polyether ether ketone (PEEK).

5. A composite material as claimed in any one of the preceding claims, wherein the composite material comprises 30 to 48 volume % polarylether ketone.

6. A composite material as claimed in any one of the preceding claims, wherein the volume ratio of reinforcement fibre to polarylether ketone is 1.1 - 1.5 : 1.

7. A composite material as claimed in any one of the preceding claims, which comprises: 52 to 58 volume % carbon fibre, 37 to 48 volume % PEEK, and up to 6 weight % barium sulphate.

8. A sheet or tape comprising the composite material as claimed in any one of the preceding claims.

9. A medical device comprising a composite material as claimed in any one of claims 1 to 7.

10. A medical device as claimed in claim 9, which is an implantable medical device.

11. A medical device as claimed in claim 9 or 10, which is selected from a trauma plate or a fracture plate.

12. A method or manufacturing a device, said method comprising: compression moulding layers of composite material together to form a body portion of the device, wherein at least one layer comprises a composite material as claimed in any one of claims 1 to 7.

## Patentansprüche

1. Verbundwerkstoff, umfassend
Polyaryletherketon,
Verstärkungsfaser,
und Kontrastmittel, wobei das Kontrastmittel Barium umfasst und
wobei die Verstärkungsfaser in einer Menge von 52 bis 58 Vol.-% bezogen auf das Gesamtvolumen des Verbundwerkstoffs vorhanden ist.

2. Verbundwerkstoff nach Anspruch 1, wobei das Kontrastmittel Bariumsulfat ist und in einer Menge von 3 bis 15 Gew.-% vorhanden ist.

3. Verbundwerkstoff nach einem der vorstehenden Ansprüche, wobei die Verstärkungsfaser Kohlenstofffaser ist.

4. Verbundwerkstoff nach einem der vorstehenden Ansprüche, wobei das Polyaryletherketon Polyetheretherketon (PEEK) ist.

5. Verbundwerkstoff nach einem der vorstehenden Ansprüche, wobei der Verbundwerkstoff zu 30 bis 48 Vol.-% Polaryletherketon umfasst.

6. Verbundwerkstoff nach einem der vorstehenden Ansprüche, wobei das Volumenverhältnis von Verstärkungsfaser zu Polaryletherketon 1,1-1,5 : 1 beträgt.

7. Verbundwerkstoff nach einem der vorstehenden Ansprüche, der umfasst:
zu 52 bis 58 Vol.-% Kohlenstofffaser,
zu 37 bis 48 Vol.-% PEEK und bis zu 6 Gew.-% Bariumsulfat.

8. Bogen oder Band, umfassend den Verbundwerkstoff nach einem der vorstehenden Ansprüche.

9. Medizinische Vorrichtung, umfassend einen Verbundwerkstoff nach einem der Ansprüche 1 bis 7.

10. Medizinische Vorrichtung nach Anspruch 9, die eine implantierbare medizinische Vorrichtung ist.

11. Medizinische Vorrichtung nach Anspruch 9 oder 10, die aus einer Traumaplatte oder einer Frakturplatte ausgewählt ist.

12. Verfahren oder Herstellen einer Vorrichtung, das Verfahren umfassend: Formpressen von Schichten aus Verbundwerkstoff zusammen, um einen Körperabschnitt der Vorrichtung auszubilden, wobei mindestens eine Schicht einen Verbundwerkstoff nach einem der Ansprüche 1 bis 7 umfasst.

## Revendications

1. Matériau composite comprenant
polyaryléthercétone,
une fibre de renfort,
et agent de contraste, dans lequel l'agent de contraste comprend du baryum, et
dans lequel la fibre de renfort est présente en une quantité de 52 à 58 % en volume sur la base du volume total du matériau composite.

2. Matériau composite selon la revendication 1, dans lequel l'agent de contraste est sulfate de baryum, et étant présent en une quantité de 3 à 15 % en poids.

3. Matériau composite selon l'une quelconque des revendications précédentes, dans lequel la fibre de renfort est la fibre de carbone.

4. Matériau composite selon l'une quelconque des revendications précédentes, dans lequel le polyaryle-éther-cétone est un polyéther-éther-cétone (PEEK).

5. Matériau composite selon l'une quelconque des revendications précédentes, dans lequel le matériau composite comprend 30 à 48 % en volume de polaryléther cétone.

6. Matériau composite selon l'une quelconque des revendications précédentes, dans lequel le rapport volumique de la fibre de renfort au polyaryléther cétone va de 1,1 à 1,5 : 1.

7. Matériau composite selon l'une quelconque des revendications précédentes, qui comprend :
52 à 58 % en volume de fibre de carbone,
37 à 48 % en volume de PEEK, et jusqu'à 6 % en poids de sulfate de baryum.

8. Feuille ou ruban adhésif comprenant le matériau composite selon l'une quelconque des revendications précédentes.

9. Dispositif médical comprenant un matériau composite selon l'une quelconque des revendications 1 à 7.

10. Dispositif médical selon la revendication 9, qui est un dispositif médical implantable.

11. Dispositif médical selon la revendication 9 ou 10, qui est choisi parmi une plaque pour traumatisme ou une plaque pour fracture.

12. Procédé ou fabrication d'un dispositif, ledit procédé comprenant : le moulage par compression de couches de matériau composite ensemble pour former une partie de corps du dispositif, dans lequel au moins une couche comprend un matériau composite selon l'une quelconque des revendications 1 à 7.
